Europäisches Patentamt

European Patent Office

Office européen des brevets

(11) Numéro de publication : **0 024 971**
**B1**

(12) **FASCICULE DE BREVET EUROPÉEN**

(45) Date de publication du fascicule du brevet :
15.12.82

(21) Numéro de dépôt : 80401164.1

(22) Date de dépôt : 07.08.80

(51) Int. Cl.³ : **C 07 C 53/126, C 07 C 53/16,**
**C 07 C 53/18, C 07 C 53/19,**
**C 07 C 53/21, C 07 C 51/41**

(54) Carboxylates de nickel, leur préparation, leur utilisation dans une composition catalytique, utilisation de cette composition pour l'oligomérisation de monooléfines.

(30) Priorité : 03.09.79 FR 7922153

(43) Date de publication de la demande :
11.03.81 (Bulletin 81/10)

(45) Mention de la délivrance du brevet :
15.12.82 Bulletin 82/50

(84) Etats contractants désignés :
BE DE GB IT NL SE

(56) Documents cités :
FR A 2 264 082

(73) Titulaire : **INSTITUT FRANCAIS DU PETROLE**
**4, Avenue de Bois-Préau**
**F-92502 Rueil-Malmaison (FR)**

(72) Inventeur : **Le Pennec, Dominique**
**16, rue du Clos des Bourgognes**
**78910 Orgerus (FR)**
Inventeur : **Commereuc, Dominique**
**32, rue Abel Vacher**
**F-92190 Meudon (FR)**
Inventeur : **Chauvin, Yves**
**64, avenue du Général Leclerc**
**F-78230 Le Pecq (FR)**

# Carboxylates de nickel, leur préparation, leur utilisation dans une composition catalytique, utilisation de cette composition pour l'oligomérisation de monooléfines

La présente invention concerne de nouveaux composés du nickel bivalent, leur préparation et leur utilisation comme composants de formules catalytiques pour l'oligomérisation des oléfines.

Les sels minéraux du nickel bivalent ont été abondamment décrits et étudiés. Ils sont généralement solubles en milieu aqueux et peu ou pas solubles en milieu hydrocarboné. Au contraire, les sels de nickel bivalent d'acides carboxyliques de formule $(RCOO)_2Ni$, où R est un radical hydrocarbyle substitué ou non, sont souvent solubles, parfois de façon substantielle dans les milieux hydrocarbonés tout au moins lorsque le radical R renferme un nombre suffisant d'atomes de carbone. Cette propriété est fréquemment recherchée pour l'utilisation de ces sels de nickel en tant que catalyseurs homogènes, seuls ou associés à des acides de Lewis notamment les composés d'alkylaluminium. En effet, l'emploi en catalyse de sels de nickel insolubles se heurte à plusieurs difficultés connues, notamment activité souvent plus faible qu'avec les sels solubles et difficultés de mise en œuvre, en particulier dans les opérations industrielles en continu où l'on doit injecter des quantités précises et particulièrement faibles de catalyseur. C'est pourquoi on a déjà proposé de préparer des catalyseurs solubles d'oligomérisation, notamment de dimérisation ou de codimérisation de monooléfines par mise en réaction des carboxylates de nickel avec les halogénures d'hydrocarbylaluminium. La mise en œuvre de ces catalyseurs se heurte toutefois au fait que, dans les opérations en continu, on observe une activité souvent plus faible que dans les opérations par charges séparées et que cette activité a même tendance à décroître au cours du temps. Ce phénomène n'a pu encore trouver d'explication satisfaisante.

L'objet de la présente invention est précisément de décrire de nouveaux composés mixtes du nickel, solubles en milieu hydrocarboné qui, utilisés avec les halogénures d'hydrocarbylaluminium dans les opérations d'oligomérisation, présentent une activité catalytique accrue et une stabilité améliorée, en particulier dans les opérations en continu. Cette activité est même supérieure à celle que l'on obtient par l'emploi conjoint d'un carboxylate de nickel, d'acide halogénoacétique et d'un halogénure d'hydrocarbylaluminium comme décrit dans la demande française n° 78-35011. Ces nouveaux composés se sont également révélés utiles, en association avec des composés d'alkylaluminium, comme catalyseurs de polymérisation de dioléfines.

Ces nouveaux composés mixtes du nickel répondent à la formule générale $(R_1COO) (R_2COO)Ni$ où $R_1$ est un reste hydrocarbyle, par exemple alkyle, cycloalkyle, alkényle, aryle, aralkyle ou alkaryle, contenant au moins 5 atomes de carbone, de préférence un reste hydrocarbyle de 5 à 20 atomes de carbone, ce reste pouvant être substitué par au moins un groupe hydroxy, et $R_2$ est un reste halogénoalkyle renfermant de 1 à 3 atomes de carbone, de formule $C_mH_pX_q$ où $m = 1, 2$ ou $3$, $p$ est égal à zéro ou à un nombre entier et $q$ est un nombre entier, avec la condition que $p + q = 2m + 1$. De préférence, $R_2$ est un reste halogénométhyle $CX_nH_{3-n}$ où X est le fluor, le chlore, le brome ou l'iode avec $n$ entier de 1 à 3.

La haute activité des compositions catalytiques obtenues à partir des composés du nickel ci-dessus et des halogénures d'hydrocarbylaluminium est d'autant plus inattendue que les compositions catalytiques obtenues à partir des bis (halogénoacétates) de nickel et des halogénures d'hydrocarbylaluminium n'ont elles-mêmes qu'une activité catalytique comparativement réduite, due probablement à l'insolubilité de ces composés du nickel dans les milieux hydrocarbonés. Les nouveaux composés du nickel de l'invention présentent au contraire une solubilité importante dans les milieux hydrocarbonés, c'est-à-dire une solubilité d'au moins 0,1 g par litre dans les conditions d'utilisation.

Les composés mixtes $(R_1COO) (R_2COO)Ni$ peuvent être préparés en faisant agir le mélange en quantités sensiblement équimolaires des deux acides $R_1COOH$ et $R_2COOH$ sur du nickel métallique ou sur un oxyde, hydroxyde ou carbonate de nickel. Ils peuvent aussi être préparés en milieu aqueux en mettant en contact les sels de métaux alcalins (y compris les sels d'ammonium), solubles dans l'eau, des deux acides $R_1COOH$ et $R_2COOH$ en proportions de préférence sensiblement équimolaires, avec un sel de nickel bivalent soluble dans l'eau. A titre d'exemples de sel minéral convenable, on peut citer le chlorure de nickel, le bromure de nickel, l'iodure de nickel, le sulfate de nickel ou le nitrate de nickel et leurs hydrates. Les sels alcalins peuvent être formés in situ en utilisant les acides organiques et en opérant en présence d'une base fournissant le métal alcalin.

La réaction est effectuée de préférence dans un milieu biphasique liquide comprenant une phase aqueuse et une phase organique non miscible de manière notable, constituée par des hydrocarbures ou des mélanges d'hydrocarbures aliphatiques, cycloaliphatiques ou aromatiques, ou par des hydrocarbures halogénés, par exemple des hydrocarbures chlorés, fluorés ou bromés. On peut par exemple utiliser le pentane, l'heptane, l'éther de pétrole, le white spirit, le cyclohexane, le toluène, les xylènes, l'éthylbenzène, le chlorure de méthylène, le chloroforme. On élimine ensuite la phase aqueuse et le sel de métal alcalin formé par double décomposition.

On peut aussi, dans un autre mode opératoire, préparer le mélange des sels alcalins des deux acides $R_1COOH$ et $R_2COOH$ dans un alcool inférieur ayant, par exemple, de 1 à 4 atomes de carbone, par exemple le méthanol ou l'éthanol, puis introduire dans un deuxième temps le sel de nickel. Après réaction, l'alcool est éliminé au moins en majeure partie par distillation et est remplacé par un hydrocarbure ou un hydrocarbure halogéné tel que décrit précédemment. On peut alors éliminer les sels minéraux par filtration.

On peut encore, dans une variante de ce mode opératoire, préparer le mélange des sels de métal alcalin des deux acides $R_1COOH$ et $R_2COOH$ dans un milieu mixte comprenant un alcool, par exemple le méthanol ou l'éthanol, et un hydrocarbure ou un hydrocarbure halogéné tel que décrit précédemment. La composition du mélange alcool-hydrocarbure peut être variable, par exemple identique à celle de l'azéotrope formé éventuellement par les deux solvants. On peut ensuite éliminer l'alcool ou le mélange des solvants.

Un autre mode opératoire, qui conduit également aux composés mixtes $(R_1COO)$ $(R_2COO)Ni$, consiste à préparer séparément, par des méthodes connues, les deux composés $(R_1COO)_2Ni$ et $(R_2COO)_2Ni$, puis à mettre ces deux composés en réaction dans des proportions sensiblement équimolaires dans un milieu liquide comprenant un solvant polaire, par exemple, l'eau ou un alcool et un solvant non polaire, par exemple un hydrocarbure ou un hydrocarbure halogéné et à éliminer ensuite le solvant polaire par distillation.

Quand on part d'acides libres, la base fournissant le métal alcalin est introduite de préférence en quantité sensiblement stœchiométrique par rapport aux deux acides sous la forme par exemple de soude ou de potasse, des carbonates ou des hydrogénocarbonates correspondants, ou d'ammoniaque.

Cependant on peut avoir intérêt, pour faciliter certaines opérations d'isolement du sel de nickel, d'utiliser un léger défaut, par exemple de 5 à 30 %, de l'un des réactifs, par rapport à la stœchiométrie, par exemple un défaut de la base.

On peut utiliser les acides ou leurs sels de proportion stœchiométrique par rapport au sel de nickel, mais on peut s'écarter de cette valeur. Ainsi l'emploi d'un excès, par exemple 5 à 20 % molaire, de sel de nickel est favorable à l'obtention d'un produit pur.

L'ordre d'introduction des réactifs n'est pas critique. Cependant il est préférable de mélanger en premier lieu les deux acides $R_1COOH$ et $R_2COOH$ avec la base en milieu polaire de façon à obtenir les sels, puis d'introduire le sel de nickel et la phase d'hydrocarbure ou d'hydrocarbure halogéné.

La quantité d'eau ou d'alcool mise en œuvre n'est pas non plus critique, mais selon l'invention les meilleurs résultats sont obtenus avec la quantité minimale nécessaire à la solubilisation des réactifs à la température de réaction. La quantité de phase hydrocarbonée mise en œuvre doit être suffisante pour assurer la solubilisation du sel mixte et est donc variable avec la nature des réactifs mis en jeu ; une quantité préférable sera comprise entre 2 et 100 fois la quantité d'eau ou d'alcool introduite.

La température de réaction est choisie en fonction des facilités opératoires ; un mode préféré consiste toutefois à porter le mélange réactionnel à reflux, à pression atmosphérique, supérieure ou inférieure, jusqu'à coloration stable de la phase organique ; cette opération peut demander par exemple entre 5 minutes et 5 heures selon la température de reflux.

La phase polaire, contenant les sels minéraux peut être éliminée par décantation et soutirage, mais on a observé, et ceci constitue un autre élément de la présente invention, que les vitesses de réaction et des rendements particulièrement élevés en composés mixtes de nickel sont obtenus par distillation hétéroazéotropique du solvant polaire. Cette distillation est de préférence poursuivie jusqu'à épuisement total de la phase polaire. Dans ce cas, les sels insolubles formés sont éliminés par décantation ou filtration. Le solvant hydrocarbure ou hydrocarbure chloré peut être évaporé ou distillé et le composé du nickel peut être isolé sous forme d'un solide vert généralement amorphe ; pour certains usages, toutefois, on peut l'utiliser directement en solution.

Un autre objet de la présente invention est l'utilisation de ces sels mixtes de nickel bivalent comme composants d'une nouvelle formule catalytique utilisable pour l'oligomérisation et, en particulier, la dimérisation et la trimérisation des monooléfines. Cette nouvelle formule concerne plus précisément les combinaisons obtenues par mise en contact, dans un ordre quelconque, d'au moins un sel mixte de nickel avec au moins un halogénure d'hydrocarbylaluminium.

On peut utiliser par exemple, à titre individuel ou en mélange, sans que la liste soit limitative l'éthyl-2-butyrate trifluoroacétate de nickel, l'éthyl-2-butyrate trichloroacétate de nickel, le diméthyl-3, 3-butyrate trifluoroacétate de nickel, le diméthyl-3,3-butyrate trichloroacétate de nickel, le méthyl-4-valérate trifluoroacétate de nickel, l'hexanoate trichloroacétate de nickel, l'heptanoate trifluoroacétate de nickel, l'heptanoate trichloroacétate de nickel, l'heptanoate tribromoacétate de nickel, l'heptanoate triiodoacétate de nickel, l'éthyl-2-hexanoate trifluoroacétate de nickel, l'éthyl-2-hexanoate monofluoroacétate de nickel, l'éthyl-2-hexanoate trichloroacétate de nickel, l'éthyl-2-hexanoate dichloroacétate de nickel, l'éthyl-2-hexanoate monochloroacétate de nickel, l'éthyl-2-hexanoate tribromoacétate de nickel, l'éthyl-2-hexanoate triiodoacétate de nickel, l'octoate trifluoroacétate de nickel, l'octoate trichloroacétate de nickel, le décanoate trifluoroacétate de nickel, le décanoate trichloroacétate de nickel, le myristate trifluoroacétate de nickel, le palmitate trifluoroacétate de nickel, le dodecylbenzoate trifluoroacétate de nickel, le diisopropylsalicylate trichloroacétate de nickel, le myristate pentafluoropropionate de nickel et l'éthyl-2 hexanoate heptafluorobutyrate de nickel.

Les composés d'halogénures d'hydrocarbylaluminium préférés répondent à la formule générale $Al_2R_xY_y$ où R représente un groupement hydrocarboné contenant jusqu'à 12 atomes de carbone, ou davantage, tels que alkyle, aryle, aralkyle, alkaryle, cycloalkyle, Y représente un halogène fluor, chlore, brome ou iode et x et y ont chacun une valeur de 2, 3 ou 4 avec $x + y = 6$. Comme exemples de tels

3

**0 024 971**

composés, on peut mentionner le sesquichlorure d'éthylaluminium, le dichloroéthylaluminium, le dichloroisobutylaluminium, le chlorodiéthylaluminium ou leurs mélanges. Des exemples de compositions catalytiques sont constitués par l'un quelconque des composés mixtes de nickel de la liste ci-dessus et l'un quelconque des composés de l'aluminium mentionnés.

L'invention a aussi pour objet un procédé d'oligomérisation de monooléfines en présence du système catalytique ci-dessus défini à une température de − 20 °C à + 80 °C dans des conditions de pression telles que les réactifs soient maintenus au moins en majorité en phase liquide ou en phase condensée.

Les monooléfines susceptibles d'être dimérisées ou oligomérisées sont, par exemple, l'éthylène, le propylène, les n-butènes, les n-pentènes, purs ou sous forme de mélanges tels qu'issus des procédés de synthèse comme le cracking catalytique ou le cracking à la vapeur. Elles peuvent être cooliglomérisées entre elles ou avec l'isobutène, l'éthylène avec le propylène et les n-butènes, le propylène avec les n-butènes, les n-butènes avec l'isobutène, par exemple.

La concentration, exprimée en nickel, de la composition catalytique dans la phase liquide de réaction d'oligomérisation est normalement comprise entre 5 et 500 parties par million en poids. Le rapport molaire de l'halogénure d'hydrocarbylaluminium au composé du nickel est normalement compris entre : 1 : 1 et 50 : 1 et plus avantageusement compris entre 2 : 1 et 20 : 1.

Le procédé peut être mis en œuvre par charges séparées mais présente un maximum d'avantages en cas d'opération continue. Dans ce cas, on peut utiliser un réacteur à un ou plusieurs étages de réaction en série, la charge oléfinique et/ou les constituants du catalyseur étant introduits en continu soit dans le premier étage soit dans le premier et un quelconque des étages. On peut également n'introduire dans le second et/ou le « n »ième étage que des quantités complémentaires d'un seul des constituants du mélange catalytique.

A la sortie du réacteur le catalyseur peut être désactivé de manière connue, par exemple à l'aide d'ammoniac et/ou d'une solution aqueuse de soude et/ou d'une solution aqueuse d'acide sulfurique. Les oléfines non converties et les alcanes éventuellement présents sont ensuite séparés des oligomères par distillation.

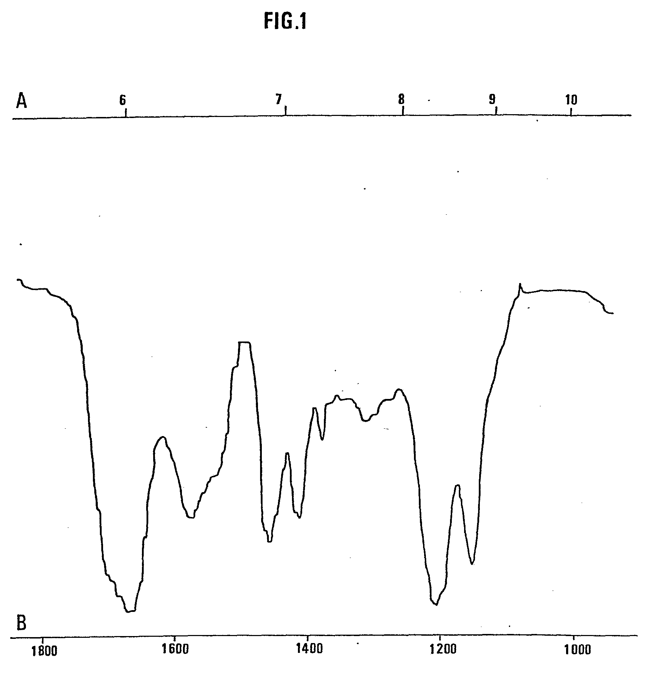

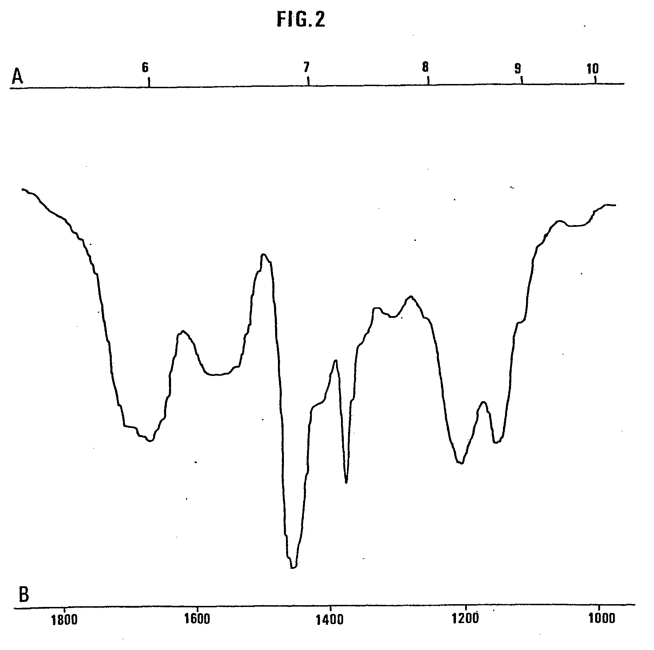

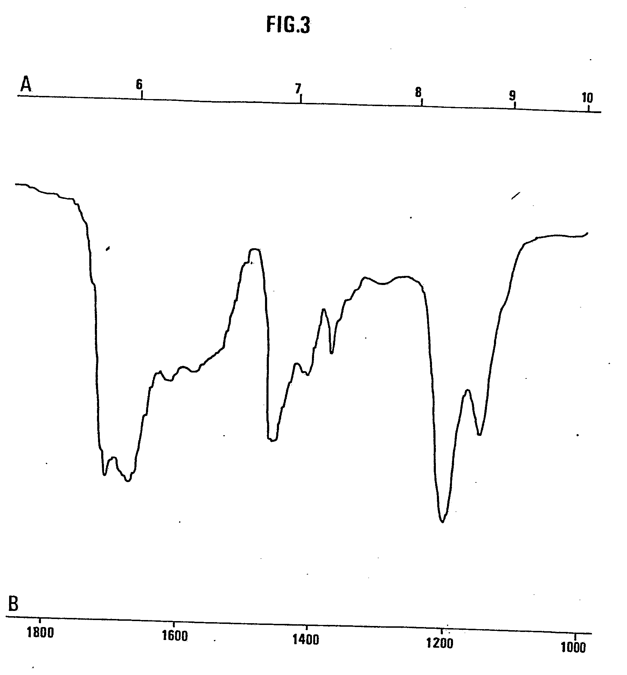

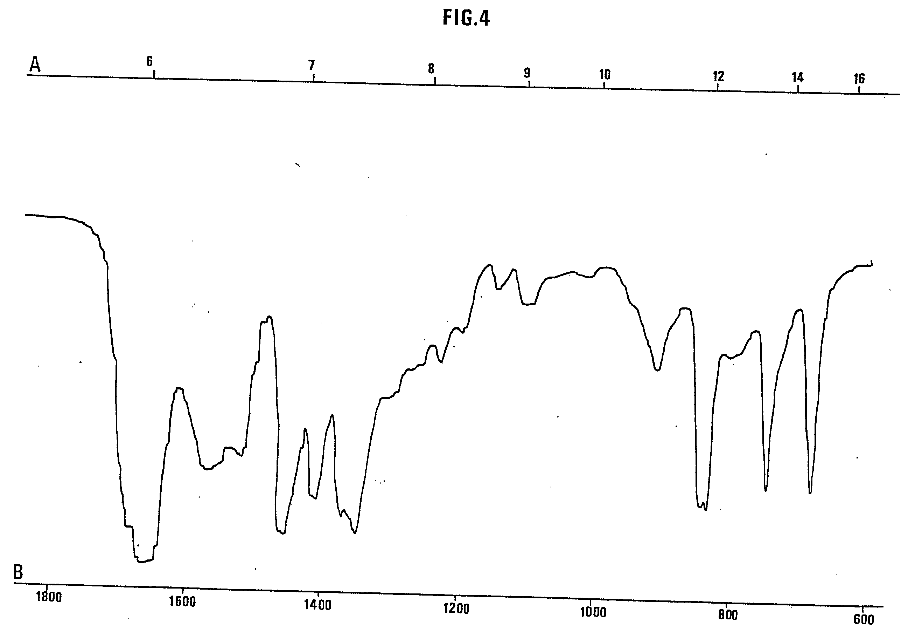

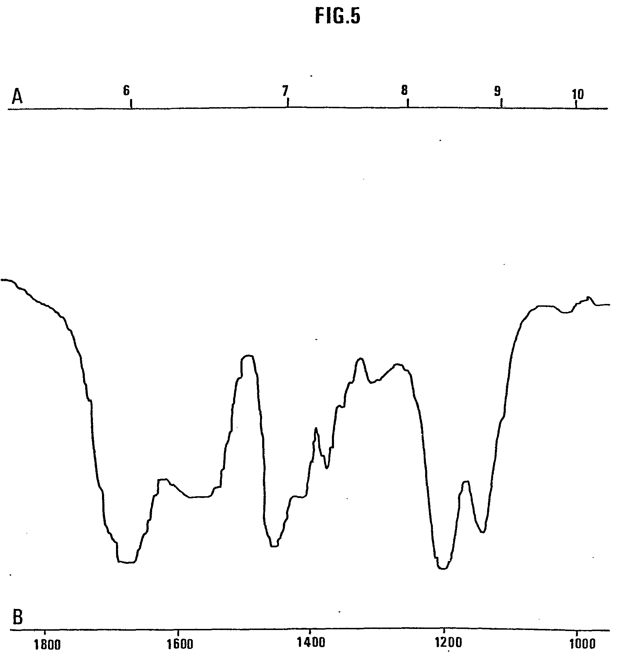

Les exemples suivants illustrent l'invention et ne sont aucunement limitatifs. La portion caractéristique du spectre d'absorption infrarouge des composés obtenus aux exemples 1 à 5 est donnée sur les figures 1 à 5 (nujol, KBr). A est la longueur d'onde en microns et B le nombre d'ondes en cm⁻¹.

### Exemple 1

Dans un ballon en verre de 500 cm³ équipé d'un barreau d'agitation magnétique et d'un dispositif pour distillation hétéroazéotropique, on introduit successivement : 14,4 g d'acide éthyl-2-hexanoïque (0,1 mole), 200 cm³ d'heptane, 11,4 g d'acide trifluoroacétique (0,1 mole), 10,6 g de carbonate de sodium, 23,7 g de chlorure de nickel hexahydraté (0,1 mole) et 25 cm³ d'eau. On porte l'ensemble à reflux et l'eau est éliminée par distillation azéotropique. Après filtration du précipité insoluble renfermant du NaCl, qui est lavé à l'heptane, la phase organique est évaporée sous vide. Il subsiste un solide vert intense, identifié par son analyse élémentaire et par son spectre infrarouge comme étant l'éthyl-2-hexanoate trifluoroacétate de nickel. Analyse élémentaire : calculé pour $C_{10}H_{15}O_4F_3Ni$ : C = 38,1 ; H = 4,8 ; Ni = 18,7 ; trouvé : C = 38,7 ; H = 5,6 ; Ni = 16,9 %. Spectre infrarouge bandes caractéristiques à : 1 670 cm⁻¹ ($CF_3COO$), 1 575 et 1 410 cm⁻¹ ($C_7H_{15}COO$), 1 205 et 1 150 cm⁻¹ ($CF_3$). On a recueilli 29,5 g de sel mixte, soit un rendement de 93,1 %.

Le fait qu'il s'agit bien d'un composé mixte et non d'un mélange de trifluoroacétate de nickel et d'éthyl-2-hexanoate de nickel est démontré comme suit : le trifluoroacétate de nickel est insoluble dans les hydrocarbures ; s'il s'était formé, il aurait donc été éliminé à l'état solide et le rendement en composé soluble dans les hydrocarbures aurait été bien plus faible.

### Exemple 2

On opère comme dans l'exemple 1 en introduisant successivement : 80 cm³ d'heptane, 5 cm³ d'eau, 0,91 g d'acide trifluoroacétique (8 × 10⁻³ mole), 1,04 g d'acide heptanoïque (8 × 10⁻³ mole), 0,64 g de soude (1,6 × 10⁻² mole) et enfin 2,81 g de sulfate de nickel heptahydraté (10⁻² mole). On obtient un solide vert (poids : 2,23 g, rendement : 88,8 %) identifié comme étant l'heptanoate trifluoroacétate de nickel. Analyse élémentaire : calculé pour $C_9H_{13}O_4F_3Ni$ : C = 35,9 ; H = 4,3 ; F = 18,9 ; Ni = 19,6 ; trouvé : C = 35,6 ; H = 4,5 ; F = 18,7 ; Ni = 19,1 %. Spectre infrarouge : bandes caractéristiques à : 1 675 cm⁻¹ ($CF_3COO$), 1 570 cm⁻¹ ($C_6H_{13}COO$), 1 205 et 1 150 cm⁻¹ ($CF_3$).

### Exemple 3

En utilisant le mode opératoire de l'exemple 1, on met en réaction : 80 cm³ d'heptane, 5 cm³ d'eau, 0,91 g d'acide trifluoroacétique (8 × 10⁻³ mole), 1,71 g d'acide myristique (8 × 10⁻³ mole), 0,64 g de soude (1,6 × 10⁻² mole) et 2,81 g de sulfate de nickel heptahydraté (10⁻² mole). On obtient 2,80 g d'un solide vert identifié comme étant le myristate trifluoroacétate de nickel (rendement : 90,9 %). Analyse

4

élémentaire : calculé pour $C_{15}H_{25}O_4F_3Ni$ : C = 46,8 ; H = 6,5 ; F = 14,8 ; Ni = 15,3 ; trouvé : C = 47,7 ; H = 7,1 ; F = 14,4 ; Ni = 14,8 %. Spectre infrarouge : bandes caractéristiques à : 1 675 cm$^{-1}$ (CF$_3$COO), 1 580 et 1 410 cm$^{-1}$ (C$_{12}$H$_{25}$COO), 1 205 et 1 150 cm$^{-1}$ (CF$_3$).

Exemple 4

En utilisant le mode opératoire de l'exemple 1, on met en réaction : 100 cm$^3$ d'heptane, 25 cm$^3$ d'eau, 5,75 g d'acide éthyl-2-hexanoïque (4 × 10$^{-2}$ mole), 6,55 g d'acide trichloroacétique (4 × 10$^{-2}$ mole), 3,20 g de soude (8 × 10$^{-2}$ mole) et 14,05 g de sulfate de nickel heptahydraté (5 × 10$^{-2}$ mole). On obtient 10,8 g d'un solide vert identifié comme étant l'éthyl-2-hexanoate trichloroacétate de nickel (rendement : 74,2 %). Analyse élémentaire : calculé pour $C_{10}H_{15}O_4Cl_3Ni$ : C = 32,9 ; H = 4,1 ; Ni = 16,2 ; trouvé : C = 37,5 ; H = 5,7 ; Ni = 15,2 %. Spectre infrarouge : bandes caractéristiques à : 1 655 cm$^{-1}$ (CCl$_3$COO), 1 570 et 1 410 cm$^{-1}$ (C$_7$H$_{15}$COO), 845 et 835 cm$^{-1}$ (CCl$_3$).

Exemple 5

En utilisant le mode opératoire de l'exemple 1, on met en réaction : 80 cm$^3$ d'heptane, 5 cm$^3$ d'eau, 0,91 g d'acide trifluoroacétique (8 × 10$^{-3}$ mole), 0,93 g d'acide méthyl-4-valérique (8 × 10$^{-3}$ mole), 0,64 g de soude (1,6 × 10$^{-2}$ mole), puis 2,81 g de sulfate de nickel heptahydraté (10$^{-2}$ mole), On obtient 0,6 g d'un solide vert identifié comme étant le méthyl-4-valérate trifluoroacétate de nickel (rendement : 26 %). Analyse élémentaire : calculé pour $C_8H_{11}O_4F_3Ni$ : C = 33,4 ; H = 3,8 ; Ni = 20,5 ; trouvé : C = 33,3 ; H = 3,9 ; Ni = 20,1 %. Spectre infrarouge : bandes caractéristiques à 1 675 cm$^{-1}$ (CF$_3$COO), 1 570 et 1 410 cm$^{-1}$ (C$_5$H$_{11}$COO), 1 200 et 1 145 cm$^{-1}$ (CF$_3$).

Exemple 6

Un réacteur d'oligomérisation est constitué de deux étages de réaction montés en série et composés, chacun, d'un réacteur cylindrique en acier à double enveloppe, thermorégulé par circulation d'eau, d'un volume utile de 0,25 litre.

On introduit en continu dans le réacteur constituant le premier étage 98 g/h d'une coupe C$_4$ dont la composition est la suivante :

| | |
|---|---|
| — propane : | 1,1 % poids |
| — isobutane : | 6,7 % poids |
| — n-butane : | 23,0 % poids |
| — butène-1 : | 5,2 % poids |
| — butène-2-trans : | 46,4 % poids |
| — butène-2-cis : | 17,6 % poids |

0,031 g/h d'éthyl-2-hexanoate trifluoroacétate de nickel préparé dans l'exemple 1, sous forme d'une solution dans l'heptane, et 0,194 g/h de dichloroéthylaluminium en solution dans l'heptane. On maintient la pression des réacteurs à 5 bars par soutirage en continu du produit de réaction, et la température à 42 °C au moyen de la circulation d'eau.

Après 4 heures de marche, on atteint un régime stationnaire stable correspondant à une conversion des butènes en oligomères de 66 % dans le premier étage et de 75 % à la sortie du deuxième étage. Les oligomères comprennent 85 % de dimères (n-octènes, méthylheptènes et diméthylhexènes), 12 % de trimères et 3 % de tétramères.

L'activité du système catalytique, exprimée par la constante de vitesse k (mol$^{-1}$ · l · h$^{-1}$) définie par la relation : $V = kC^2$, où V est la vitesse de réaction (mol · l$^{-1}$ · h$^{-1}$) et C est la concentration stationnaire en butènes (mol · l$^{-1}$) dans les étages, est égale à :

— k (1$^{er}$ étage) = 0,423 mol$^{-1}$ · l · h$^{-1}$
— k (2$^e$ étage) = 0,106 mol$^{-1}$ · l · h$^{-1}$

Exemple 7

Dans le même appareillage que celui de l'exemple 6, on introduit en continu 83 g/h de la même coupe C$_4$, 0,036 g/h d'éthyl-2-hexanoate trichloroacétate de nickel préparé dans l'exemple 4 sous forme d'une solution dans l'heptane, et 0,194 g/h de dichloroéthylaluminium en solution dans l'heptane.

Après 4 heures de marche, on atteint un régime stationnaire stable correspondant à une conversion des butènes de 64 % dans le premier étage et de 77 % dans le deuxième étage.

L'activité du système catalytique exprimée comme dans l'exemple 6, est de :

— k (1$^{er}$ étage) = 0,307 mol$^{-1}$ · l · h$^{-1}$
— k (2$^e$ étage) = 0,152 mol$^{-1}$ · l · h$^{-1}$

# 0 024 971

## Exemple 8

Cet exemple est donné à titre comparatif pour montrer les avantages de l'invention.

Dans le même appareillage que celui de l'exemple 6, on introduit en continu 82 g/h de la même coupe $C_4$, 0,052 g/h d'un carboxylate de nickel à 11 % en poids de nickel, mélangé avec 0,011 g/h d'acide trifluoroacétique dans l'heptane, et 0,194 g/h de dichloroéthylaluminium en solution dans l'heptane.

La quantité de nickel introduite, exprimée en g/h de Ni métallique, est identique à celle de l'essai décrit dans l'exemple 6, ce qui permet une comparaison directe des résultats au moyen de la constante k.

Le régime stationnaire atteint après 4 heures de marche correspond à une conversion de 57 % dans le premier étage et de 68 % dans le deuxième étage. L'activité de ce système catalytique est donc notablement inférieure à celle observée dans l'exemple 6 :

— k (1$^{er}$ étage) = 0,193 mol$^{-1} \cdot$ l $\cdot$ h$^{-1}$
— k (2$^e$ étage) = 0,067 mol$^{-1} \cdot$ l $\cdot$ h$^{-1}$

## Exemple 9

Dans un ballon en verre de 500 cm$^3$ équipé d'un barreau d'agitation magnétique et d'un dispositif de reflux, on introduit successivement : 14,4 g d'acide éthyl-2-hexanoïque (0,1 mole), 100 cm$^3$ de méthanol, 11,4 g d'acide trifluoroacétique (0,1 mole) et 8,0 g d'hydroxyde de sodium (0,2 mole). Le mélange est porté à reflux, puis on introduit 35,1 g (0,125 mole) de sulfate de nickel heptahydraté. On porte de nouveau à reflux. On évapore le méthanol au moyen d'un évaporateur rotatif et le résidu est repris au toluène. Après filtration des sels minéraux insolubles, le toluène est distillé et on obtient avec un bon rendement l'éthyl-2-hexanoate trifluoroacétate de nickel. Son analyse correspond à la formule $C_{10}H_{15}O_4F_3Ni$ et il présente les bandes caractéristiques du produit de l'exemple 1.

## Exemple 10

Dans un réacteur d'oligomérisation ayant un volume utile de 35 litres, on introduit en continu 5 kg/h d'une coupe $C_3$ dont la composition est la suivante :

— Propane   : 20 % poids
— Propylène : 80 % poids

0,273 g/h d'éthyl-2-hexanoate trifluoroacétate de nickel sous forme d'une solution dans l'isooctane et 1,650 g/h de dichloroéthylaluminium en solution dans l'isooctane. On maintient la pression à 15 bars par soutirage du produit de réaction, et la température à 42 °C par une circulation d'eau. La conversion du propylène en oligomères, en régime stationnaire, est de 90 % et les oligomères obtenus renferment 85 % de dimères (n-hexènes, méthylpentènes et diméthylbutènes), 12 % de trimères et 3 % de tétramères.

## Exemple 11

Dans un ballon en verre de 1 litre équipé d'un barreau d'agitation magnétique et d'un dispositif de distillation, on introduit 25 g de NiO, 46 cm$^3$ d'acide trifluoroacétique et 90 cm$^3$ d'eau distillée. Le mélange est porté à reflux pendant 2 heures, puis l'excès de NiO est filtré. On obtient ainsi quantitativement une solution aqueuse de trifluoroacétate de nickel $Ni(CF_3COO)_2$. A cette solution aqueuse, on ajoute 135 g d'éthyl-2-hexanoate de nickel technique à 13 % en poids de nickel, et contenant entre autres, 10 % d'acide éthyl-2-hexanoïque libre, dissous dans 500 cm$^3$ d'heptane. On porte à reflux puis on élimine l'eau par distillation azéotropique. Après enlèvement complet de l'eau, l'heptane est éliminé par évaporation sous vide. L'éthyl-2-hexanoate trifluoroacétate de nickel est ainsi obtenu avec un rendement de 98 %. Analyse élémentaire : calculé pour $C_{10}H_{15}O_4F_3Ni$ : C = 38,1 ; H = 4,8 ; Ni = 18,7 ; trouvé : C = 38,4 ; H = 5,4 ; Ni = 17,8. Le spectre infrarouge présente les bandes caractéristiques attendues.

En résumé, les sels mixtes de l'invention sont préparés en faisant réagir une source d'ion $R_1COO$— en solution avec une source d'ion $R_2COO$— en solution et une source d'ion nickel (qui peut être commune avec les deux sources précitées) en solution. Le solvant utilisé pour la mise en solution peut être commun à toutes les sources ou des solvants distincts peuvent être utilisés. On utilise habituellement un solvant polaire, de préférence eau et/ou alcool. Simultanément ou postérieurement on ajoute un solvant hydrocarbure ou hydrocarbure halogéné et on chasse le solvant polaire, de préférence par distillation. Le sel mixte est obtenu, en solution dans l'hydrocarbure ou l'hydrocarbure halogéné.

## Revendications

1. Nouveau composé de nickel de formule générale $(R_1COO)$ $(R_2COO)Ni$, où $R_1$ est un radical

6

**0 024 971**

hydrocarbyle ou hydrocarbyle substitué par au moins un groupe hydroxy, contenant au moins 5 atomes de carbone, et $R_2$ est un reste halogénoalkyle renfermant de 1 à 3 atomes de carbone.

2. Nouveau composé de nickel selon la revendication 1, caractérisé en ce que $R_1$ est un radical alkyle et en ce que $R_2$ est un reste trifluorométhyle $CF_3$ ou trichlorométhyle $CCl_3$.

3. Nouveau composé de nickel selon la revendication 1, caractérisé en ce qu'il consiste en l'éthyl-2-hexanoate trifluoroacétate de nickel.

4. Procédé de fabrication d'un composé de nickel selon l'une des revendications 1 à 3, caractérisé par la mise en réaction de sels de métaux alcalins des deux acides $R_1COOH$ et $R_2COOH$ avec un sel de nickel bivalent, en milieu solvant.

5. Procédé selon la revendication 4, dans lequel on opère dans un milieu biphasique constitué d'une phase aqueuse et d'une phase de solvant organique non miscible notablement à l'eau, puis on distille l'eau, on sépare le sel de métal alcalin précipité et on recueille le composé de nickel en solution dans le solvant organique.

6. Procédé selon la revendication 4, dans lequel on opère dans un alcool et on chasse ensuite l'alcool par distillation, on le remplace par un hydrocarbure ou un hydrocarbure halogéné et on filtre la solution pour séparer les sels précipités de la solution du composé de nickel.

7. Procédé de fabrication d'un composé de nickel selon l'une des revendications 1 à 3, caractérisé par la mise en réaction d'un sel de nickel de formule $(R_1COO)_2Ni$ avec un sel de nickel de formule $(R_2COO)_2Ni$ dans un milieu liquide comprenant un solvant polaire, eau ou alcool, et un hydrocarbure ou un hydrocarbure halogéné, suivie de l'enlèvement du solvant polaire par distillation.

8. Procédé selon la revendication 5 ou 6, dans lequel on distille le solvant organique, l'hydrocarbure ou l'hydrocarbure halogéné pour obtenir le composé de nickel.

9. Procédé selon l'une des revendications 4 à 6 ou 8 dans lequel le composé de nickel est le chlorure, le sulfate ou le nitrate de nickel.

10. Utilisation d'un composé de nickel selon l'une des revendications 1 à 3 pour la préparation d'une composition catalytique, caractérisée en ce qu'on met en contact, dans un ordre quelconque, au moins un composé de nickel selon l'une des revendications 1 à 3, avec au moins un halogénure d'hydrocarbylaluminium.

11. Utilisation de la composition obtenue selon la revendication 10 comme catalyseur de conversion d'au moins une monooléfine en dimères, trimères ou oligomères, ladite conversion étant mise en œuvre à une température de $-20\,°C$ à $+80\,°C$, sous une pression telle que les réactifs soient maintenus au moins en majorité en phase liquide ou condensée.

## Claims

1. New nickel compound of the general formula $(R_1COO)(R_2COO)Ni$, where $R_1$ is a hydrocarbyl radical or a hydrocarbyl radical substituted with at least one hydroxy group, containing at least 5 carbon atoms, and $R_2$ is a halogenoalkyl radical comprising from 1 to 3 carbon atoms.

2. New nickel compound according to claim 1, characterized in that $R_1$ is an alkyl radical and $R_2$ is a trifluoromethyl $CF_3$ or a trichloromethyl $CCl_3$ group.

3. New nickel compound according to claim 1, characterized in that it consists of nickel 2-ethyl hexanoate trifluoracetate.

4. Process for manufacturing a nickel compound according to one of claims 1 to 3, characterized in reacting alkali metal salts of the two acids $R_1COOH$ and $R_2COOH$ with a bivalent nickel salt, in solvent medium.

5. Process according to claim 4, wherein the operation is conducted in a bi-phasic medium consisting of an aqueous phase and an organic solvent phase not substantially miscible with water, the water is then distilled off, the precipitate of alkali metal salt is separated and the nickel compound is recovered as a solution in the organic solvent.

6. Process according to claim 4, operated in an alcohol, wherein the alcohol is then removed by distillation, replaced with a hydrocarbon or a halogenated hydrocarbon and the solution is filtered for separating the settled salts from the solution of the nickel compound.

7. Process for manufacturing a nickel compound according to one of claims 1 to 3, characterized in that a nickel salt of the formula $(R_1COO)_2Ni$ is reacted with a nickel salt of the formula $(R_2COO)_2Ni$ in a liquid medium comprising a polar solvent, consisting of water or alcohol, and a hydrocarbon or a halogenated hydrocarbon, and the polar solvent is then removed by distillation.

8. Process according to claim 5 or 6, wherein the organic solvent, the hydrocarbon or the halogenated hydrocarbon is distilled off to obtain the nickel compound.

9. Process according to one of claims 4 to 6 or 8, wherein the nickel compound is nickel chloride, sulfate or nitrate.

10. The use of a nickel compound according to one of claims 1 to 3, for preparing a catalyst composition, characterized in that at least one nickel compound according to one of claims 1 to 3 is contacted in any order with at least one hydrocarbylaluminium halide.

11. The use of the composition obtained according to claim 10 as a catalyst for converting at least

7

one mono-olefin to dimers, trimers or oligomers, said conversion being effected at a temperature of − 20 °C to + 80 °C under sufficient pressure to maintain the reactants, at least in major part, in liquid or condensed phase.

**Ansprüche**

1. Neue Nickelverbindung, dadurch gekennzeichnet, daß sie die allgemeine Formel $(R_1COO)(R_2COO)Ni$ hat, wobei $R_1$ ein Kohlenwasserstoffradikal oder ein mit wenigstens einer Hydroxylgruppe substituiertes Kohlenwasserstoffradikal aus wenigstens 5 Kohlenstoffatomen, und $R_2$ ein Halogen-alkylrest aus 1 bis 3 Kohlenstoffatomen ist.

2. Neue Nickelverbindung gemäß Anspruch 1, dadurch gekennzeichnet, daß $R_1$ ein Alkylradikal ist, und daß $R_2$ ein Trifluormethyl-, $CF_3$-, oder Trichlormethyl-Rest, $CCl_3$-, ist.

3. Neue Nickelverbindung gemäß Anspruch 1, dadurch gekennzeichnet, daß sie aus dem 2-Ethylhexanoat-Trifluoracetat von Nickel besteht.

4. Verfahren zur Herstellung einer Nickelverbindung gemäß einem der Ansprüche 1 bis 3, dadurch gekennzeichnet, daß Salze von Alkalimetallen der zwei Säuren $R_1COOH$ und $R_2COOH$ mit einem zweiwertigen Nickelsalz in Lösungsmittel-Milieu zur Reaktion gebracht werden.

5. Verfahren gemäß Anspruch 4, dadurch gekennzeichnet, daß man in zweiphasigem Milieu arbeitet, das dargestellt wird aus einer wässrigen Phase und einer Phase organischen, nicht beträchtlich mit Wasser mischbaren Lösemittels, man dann das Wasser abdestilliert, man das ausgefällte Alkalimetall-Salz abtrennt, und man die Nickelverbindung in Lösung im organischen Lösungsmittel sammelt.

6. Verfahren gemäß Anspruch 4, dadurch gekennzeichnet, daß man in linem Alkohol arbeitet und man dann den Alkohol durch Destillation entfernt, man ihn durch einen Kohlenwasserstoff oder einen halogenierten Kohlenwasserstoff ersetzt, und man die Lösung filtert zum Abtrennen der gefällten Salze von der Lösung der Nickelverbindung.

7. Verfahren zur Herstellung einer Nickelverbindung gemäß einem der Ansprüche 1 bis 3, dadurch gekennzeichnet, daß man ein Nickel-Salz der Formel $(R_1COO)_2Ni$ mit einem Nickel-Salz der Formel $(R_2COO)_2Ni$ in einem flüssigen Milieu, das ein polares Lösemittel, Wasser oder Alkohol, und einen Kohlenwasserstoff oder einen halogenierten Kohlenwasserstoff umfasst, zur Reaktion bringt, gefolgt von der Entfernung des polaren Lösungsmittels durch Destillation.

8. Verfahren gemäß Anspruch 5 oder 6, dadurch gekennzeichnet, daß man das organische Lösemittel, den Kohlenwasserstoff oder halogenierten Kohlenwasserstoff, destilliert, um die Nickel-Verbindung zu erhalten.

9. Verfahren gemäß einem der Ansprüche 4 bis 6 oder 8, dadurch gekennzeichnet, daß die Nickel-Verbindung das Chlorid, das Sulfat oder das Nitrat von Nickel ist.

10. Verwendung einer Nickel-Verbindung gemäß einem der Ansprüche 1 bis 3 zur Herstellung einer katalytischen Zusammensetzung, dadurch gekennzeichnet, daß man, in irgendeiner Reihenfolge, wenigstens eine Nickel-Verbindung gemäß einem der Ansprüche 1 bis 3 mit wenigstens einem Aluminiumkohlenwasserstoffchlorid in Kontakt bringt.

11. Verwendung der gemäß Anspruch 10 erhaltenen Zusammensetzung als Katalysator zur Umwandlung wenigstens eines Mono-Olefins in Dimere, Trimere oder Oligomere, wobei die genannte Umwandlung durchgeführt wird bei einer Temperatur von − 20 °C bis + 80 °C, unter einem Druck, bei dem die Reaktionspartner wenigstens überwiegend in flüssiger oder kondensierter Phase gehalten werden.

## FIG.1

FIG.2

# FIG.3

A

6       7     8    9    10

B

1800    1600    1400    1200    1000

FIG.4

0 024 971

0 024 971

**FIG.5**

A

6      7      8      9      10

B

1800     1600     1400     1200     1000

5